# EUROPEAN PATENT APPLICATION

(11) **EP 4 613 767 A1**
(43) Date of publication of application: **10.09.2025**
(21) Application number: 23884946.7
(22) Date of filing: 31.10.2023
(51) Int. Cl.: C07K 16/28, C12N 15/13, C12N 15/63, C12N 5/10, A61K 39/395, A61P 35/00

(54) **BISPECIFIC ANTIBODY FOR GLYPICAN-3 AND USE THEREOF**

(30) Priority: 01.11.2022 CN 202211366621; 19.10.2023 CN 202311361967
(71) Applicant: Shanghai Qilu Pharmaceutical Research and Development Centre Ltd., Shanghai 201203 (CN)
(72) Inventor: LUO, Xiao, Shanghai 201203 (CN); SHI, Yingying, Shanghai 201203 (CN); LIU, Xiaofen, Shanghai 201203 (CN); JIANG, Jiahua, Shanghai 201203 (CN); CHEN, Shihao, Shanghai 201203 (CN)
(74) Representative: De Clercq & Partners
(86) International application number: PCT/CN2023/128662
(87) International publication number: WO 2024/094017

(57) **Abstract**

Provided are a GPC3 bispecific antigen binding molecule or an antigen binding fragment thereof, a derivative containing the bispecific antigen binding molecule or the antigen binding fragment thereof, and a pharmaceutical composition. In addition, further provided is the related use of the bispecific antigen binding molecule or the antigen binding fragment thereof in the treatment of cancers.

## Description

The present application claims priorities to Chinese Patent Application No. CN202211366621.2, filed with the China National Intellectual Property Administration on November 1, 2022 and entitled "BISPECIFIC ANTIBODY FOR GLYPICAN-3 AND USE THEREOF", and Chinese Patent Application No. CN202311361967.8, filed with the China National Intellectual Property Administration on October 19, 2023 and entitled "BISPECIFIC ANTIBODY FOR GLYPICAN-3 AND USE THEREOF", the contents of each of which are incorporated herein by reference in their entirety.

### TECHNICAL FIELD

The present disclosure belongs to the field of immunology and relates to a bispecific antigen-binding molecule against Glypican-3 (GPC3) and CD3. In addition, the present disclosure also relates to a polynucleotide encoding such bispecific antigen-binding molecules, a vector, and a host cell. The present disclosure further relates to a pharmaceutical composition comprising the bispecific antigen-binding molecule and related use thereof in the treatment and prevention of cancer.

### BACKGROUND

Glypican-3 is a proteoglycan anchored to the cell surface via glycosylphosphatidylinositol (GPI), belonging to the heparan sulfate proteoglycan (HSPG) family and is primarily involved in processes of regulating cell proliferation, adhesion, and migration, etc. GPC3 is highly expressed in normal embryonic tissues (including liver and placenta) but exhibits minimal or no expression in normal adult tissues (FEBS J, 2013, 280(10): 2471-6; Am J Surg Pathol, 2008, 32(3): 433-44). Researches have shown that GPC3 is specifically and highly expressed in hepatocellular carcinoma (HCC) tissues, observed in 70% to 80% of HCC patients. Compared to normal liver tissues, GPC3 expression in liver cancer is increased by an average of 21.7-fold. Studies have found that the 5-year survival rate of patients with GPC3-positive liver cancer is lower than that of GPC3-negative patients, and the prognosis of patients with low GPC3 expression is also generally better than that of patients with high GPC3 expression. Furthermore, GPC3 controls the proliferation of HCC cells by influencing signaling pathways such as Wnt and YAP, thereby influencing tumor growth and metastasis. Compared to alpha-fetoprotein, GPC3 demonstrates more significant sensitivity and specificity for early liver cancer detection, making it more practical (Eur Rev Med Pharmacol Sci, 2015, 19(19): 3655-73; Cancer Res, 1997, 57(22): 5179-84). As a result, GPC3 has emerged as an auxiliary diagnostic marker and therapeutic target for liver cancer. In addition, GPC3 expression has been observed in some other tumors, including lung cancer, squamous cell carcinoma, testicular non-seminoma, and liposarcoma (Am J Clin Pathol, 2008, 129(6): 899-906). As an innovative target, the development of antibody-based drugs against GPC3 has progressed slowly. Patent documents such as WO2004022597A1, WO2004022739A1, WO2009041062A1, and WO2014097648A1 have disclosed information about GPC3 antibodies and therapeutic methods, etc. Clinically, Codrituzumab (GC-33), a GPC3 monoclonal antibody developed by Chugai Pharmaceutical, is the first antibody targeting GPC3, the main principle of which is to kill tumor cells through antibody-dependent cell-mediated cytotoxicity (ADCC). Currently in Phase II clinical trials, Codrituzumab has shown no significant difference in overall survival and progression-free survival of patients compared to placebo. However, it has demonstrated improved prognosis in HCC patients with GPC3 overexpression.

T cell-directed killing of tumor cells has shown outstanding antitumor effects in numerous animal models and has recently achieved notable progress in the clinical treatment of cancer. However, high side effects, particularly severe cytokine release, have always been the utmost difficulty hindering the successful clinical application of T cell-based therapies. Therefore, it is crucial to develop novel T cell bispecific antibodies with high efficiency and low toxicity by leveraging the potential of T cells. To date, the only one bispecific binding molecule known in the prior art targeting both GPC3 and CD3ε simultaneously and has entering clinical is ERY974, developed by Chugai Pharmaceutical based on GC-33. Currently in Phase I clinical trials, ERY974 was associated with severe cytokine storms observed even at a low dose (0.81 µg/kg), leading to the suspension of dose escalation. Consequently, there remains an urgent need in the art to construct a GPC3/CD3 bispecific binding molecule with higher activity, lower toxicity, a broader therapeutic window, and the ability to achieve stronger pharmacodynamic effects at lower doses.

### SUMMARY

Provided herein is a bispecific antibody against GPC3 and CD3, which is capable of recruiting T cells to tumor sites via CD3 target and specifically killing tumor cells with high GPC3 expression.

The present disclosure relates to a bispecific antigen-binding molecule comprising a first antigen-binding domain that recognizes Glypican-3 (GPC3); and a second antigen-binding domain that recognizes a CD3 subunit of a T cell receptor; wherein the first antigen-binding domain comprises HCDR1, HCDR2, and HCDR3, which are derived from the heavy chain variable region as shown in SEQ ID NO: 10.

It should be noted that the divisions of CDR and FR in the variable regions of the antibody of the present disclosure are determined according to the Kabat definition. Other nomenclature and numbering systems, such as Chothia, IMGT, or AHo, are also known to those skilled in the art. Therefore, humanized antibodies comprising one or more CDRs derived from any nomenclature system, based on the antibody sequences of the present disclosure, are explicitly encompassed within the scope of the present disclosure.

In one embodiment, the first antigen-binding domain comprises a HCDR as shown in SEQ ID NOs: 11 to 13; preferably, the sequence of HCDR1 is as shown in SEQ ID NO: 11; the sequence of HCDR2 is as shown in SEQ ID NO: 12; and the sequence of HCDR3 is as shown in SEQ ID NO: 13.

In one embodiment, the second antigen-binding domain comprises HCDR1, HCDR2, and HCDR3 derived from the heavy chain variable region as shown in SEQ ID NO: 1; and further comprises LCDR1, LCDR2, and LCDR3 derived from the light chain variable region as shown in SEQ ID NO: 2.

In one embodiment, the second antigen-binding domain comprises a CDR as shown in SEQ ID NOs: 4 to 9; preferably, the sequence of HCDR1 is as shown in SEQ ID NO: 4; the sequence of HCDR2 is as shown in SEQ ID NO: 5; the sequence of HCDR3 is as shown in SEQ ID NO: 6; the sequence of LCDR1 is as shown in SEQ ID NO: 7; the sequence of LCDR2 is as shown in SEQ ID NO: 8; and the sequence of LCDR3 is as shown in SEQ ID NO: 9.

The present disclosure further relates to a bispecific antigen-binding molecule, which also has one or more of the following features: (1) the first antigen-binding domain is a nanobody, preferably a humanized camelid nanobody; (2) the bispecific antibody comprises heavy chain constant regions CH2 and CH3 and lacks a light chain constant region and/or heavy chain constant region CH1; preferably, the heavy chain constant region comprises an Fc domain or a variant Fc; more preferably, the Fc is derived from mouse or human; (3) the bispecific antigen-binding molecule comprises an Fc domain, wherein the Fc domain is an IgG Fc domain; preferably, the Fc domain is an IgG1 domain or an IgG4 domain; (4) the bispecific antigen-binding molecule comprises an Fc domain, wherein the two polypeptides constituting the Fc domain have different sequences from each other; the Knob chain of the two polypeptides constituting the Fc domain comprises a mutation of amino acid residues to tryptophan at position 366 according to the EU numbering system; the Hole chain comprises a mutation of amino acid residues to serine at position 366, a mutation of amino acid residues to alanine at position 368, and a mutation of amino acid residues to valine at position 407 according to the EU numbering system; preferably, the Knob chain comprises S354C and T366W amino acid substitutions, and the Hole chain comprises Y349C, T366S, L368A, and Y407V amino acid substitutions; more preferably, the Hole chain further comprises an H435R substitution; further preferably, the Fc fragment further comprises L234A and L235A substitutions; further preferably, the Fc fragment further comprises a K447A substitution at the last position of the C-terminus; (5) the bispecific antigen-binding molecule comprises an Fc domain, wherein the sequence of the Fc domain is as shown in SEQ ID NO: 14 and/or SEQ ID NO: 15.

In one embodiment, the bispecific antigen-binding molecule comprises a first antigen-binding domain and a second antigen-binding domain, wherein the first antigen-binding domain comprises a sequence as shown in SEQ ID NO: 16 or 18, and/or the second antigen-binding domain comprises a sequence as shown in SEQ ID NO: 17.

The present disclosure further relates to an isolated polynucleotide encoding the bispecific antigen-binding molecule or a fragment thereof as described in any one of the preceding embodiments; or a recombinant vector comprising the isolated polynucleotide; or a host cell comprising the isolated polynucleotide or recombinant vector.

The present disclosure further relates to a method for producing the bispecific antigen-binding molecule as described in any one of the preceding embodiments.

The present disclosure further relates to a pharmaceutical composition comprising: a pharmaceutically acceptable carrier, and one or more selected from the group consisting of the bispecific antigen-binding molecule, polynucleotide, recombinant vector, and/or host cell as described in any one of the preceding embodiments.

The present disclosure further relates to the use of the bispecific antigen-binding molecule or pharmaceutical composition in the manufacture of a medicament for treating and/or preventing a disease in an individual in need thereof; preferably, the disease is cancer; more preferably, the cancer is a GPC3-positive tumor; further preferably, the cancer is selected from liver cancer, lung cancer, and skin cancer.

The present disclosure further relates to a method for treating and/or preventing a disease associated with GPC3 expression in a subject, comprising administering to the subject a therapeutically and/or prophylactically effective amount of the bispecific antigen-binding molecule or pharmaceutical composition as described in any one of the preceding embodiments; preferably, the disease associated with GPC3 expression is cancer; more preferably, the cancer is selected from liver cancer, lung cancer, and skin cancer.

The present disclosure further relates to a method for preparing a pharmaceutical composition for treating and/or preventing a disease associated with GPC3 expression in a subject, comprising administering to the subject a therapeutically and/or prophylactically effective amount of the bispecific antigen-binding molecule or pharmaceutical composition as described in any one of the preceding embodiments; preferably, the disease associated with GPC3 expression is cancer; more preferably, the cancer is selected from liver cancer, lung cancer, and skin cancer.

### BRIEF DESCRIPTION OF THE DRAWINGS

The accompanying drawings further illustrate the novel features disclosed in this specification. The features and advantages disclosed in this specification will be better understood with reference to these accompanying drawings, but it should be understood that these accompanying drawings are merely illustrative of specific embodiments of the principles disclosed herein and are not intended to limit the scope of the appended claims.
FIG. 1 shows the structures of bispecific antigen-binding molecules G100 and G156.
FIG. 2 shows the binding of bispecific antigen-binding molecules G100 and G156, as well as a control antibody, to HepG2 cells naturally expressing hGPC3.
FIG. 3 shows the binding of bispecific antigen-binding molecules G100 and G156, as well as a control antibody, to Jurkat cells naturally expressing hCD3.
FIG. 4 shows the results of a T cell-dependent cellular cytotoxicity (TDCC) assay of CD3⁺ T cells against HepG2 cells naturally expressing hGPC3 mediated by bispecific antigen-binding molecules G100 and G156.
FIG. 5 shows the results of T cell activation of bispecific antigen-binding molecules G100 and G156, as well as a control antibody, on HepG2 cells naturally expressing hGPC3.
FIG. 6 shows the results of IL-2 secretion of bispecific antigen-binding molecules G100 and G156, as well as a control antibody, on HepG2 cells naturally expressing hGPC3.
FIG. 7 shows the results of IFNγ secretion of bispecific antigen-binding molecules G100 and G156, as well as a control antibody, on HepG2 cells naturally expressing hGPC3.
FIG. 8 shows the results of TNF-α secretion of bispecific antigen-binding molecules G100 and G156, as well as a control antibody, on HepG2 cells naturally expressing hGPC3.
FIG. 9 shows the effect of bispecific antibody G100 of the present disclosure on tumor growth in a subcutaneous xenograft model of human hepatocellular carcinoma HepG2 cells mixed with PBMCs.

### DETAILED DESCRIPTION OF THE INVENTION

### Terminology

All publications, patents, and patent applications mentioned in this specification are incorporated herein by reference to the same extent as if each individual publication, patent, or patent application was specifically and individually indicated to be incorporated by reference.

Before the present disclosure is described in detail below, it should be understood that the present disclosure is not limited to the particular methodology, protocols, and reagents described herein, as these may vary. It should also be understood that the terms used herein are merely for describing specific embodiments, and are not intended to limit the scope of the present disclosure. Unless otherwise defined, all technical and scientific terms used herein have the same meanings as those commonly understood by one of ordinary skill in the art to which the present disclosure belongs.

Certain embodiments disclosed herein encompass numerical ranges, and certain aspects of the present disclosure may be described in terms of ranges. Unless otherwise indicated, it should be understood that numerical ranges or descriptions in terms of ranges are merely for brevity and convenience, and should not be construed as strictly limiting the scope of the present disclosure. Accordingly, descriptions in terms of ranges should be considered to specifically disclose all possible subranges and all possible specific numerical points within the range, as if these subranges and numerical points were explicitly written herein. The above principles are equally applicable regardless of the width of the numerical values. When a range description is employed, the range includes the endpoints of the range.

As used herein, the term "antigen-binding molecule" refers in its broadest sense to a molecule that specifically binds to an antigenic determinant. Examples of antigen-binding molecules are immunoglobulins and derivatives thereof, such as fragments.

The term "bispecific" means that an antigen-binding molecule is capable of specifically binding to two different antigenic determinants. Typically, a bispecific antigen-binding molecule comprises two antigen-binding sites, each of which is specific for a different antigenic determinant. In certain embodiments, the bispecific antigen-binding molecule is capable of simultaneously binding to two antigenic determinants, particularly two antigenic determinants expressed on two different cells.

The term "antigen" refers to a substance recognized and specifically bound by an antibody or antibody-binding fragment, and in a broad sense, an antigen may include any immunogenic fragment or determinant of a selected target, including single epitopes, multiple epitopes, single domains, multiple domains, or entire extracellular domains (ECDs) or proteins. Peptides, proteins, glycoproteins, polysaccharides and lipids, portions thereof and combinations thereof may constitute antigens. Non-limiting exemplary antigens include tumor antigens, pathogen antigens, *etc.* An "antigen" may also refer to a molecule that elicits an immune response. Any form of the antigen or cells or preparations containing the antigen may be used to generate antibodies specific for the antigenic determinant. The antigen may be an isolated full-length protein, a cell surface protein (*e.g.,* immunized with cells expressing at least a portion of the antigen on their surface), a soluble protein (*e.g.,* immunized with only the ECD portion of the protein), or a protein construct (*e.g.,* an Fc antigen). The antigen may be produced in genetically modified cells. Any of the foregoing antigens may be used alone or in combination with one or more immunogenicity enhancing adjuvants known in the art. The DNA encoding the antigen may be genomic or non-genomic (*e.g.,* cDNA), and may encode at least a portion of the ECD sufficient to elicit an immunogenic response. Any vector may be used to transform cells in which the antigen is expressed, including, but not limited to, adenoviral vectors, lentiviral vectors, plasmids, and non-viral vectors such as cationic lipids.

The term "epitope" refers to a site on an antigen to which an immunoglobulin or antibody specifically binds. Epitopes may be formed from contiguous amino acids or non-contiguous amino acids juxtaposed by tertiary folding of a protein. Epitopes formed by contiguous amino acids are typically retained after exposure to denaturing solvents, while epitopes formed by tertiary folding are typically lost after treatment with denaturing solvents. An epitope typically exists in a unique spatial conformation and comprises at least 3 to 15 amino acids. Methods for determining the epitope to which a given antibody binds are well known in the art, including immunoblotting and immunoprecipitation assays. Methods for determining the spatial conformation of an epitope include techniques in the art and those described herein, such as X-ray crystallography, two-dimensional nuclear magnetic resonance, *etc.*

The terms "polypeptide", "peptide", and "protein" are used interchangeably herein to refer to polymers of amino acids of any length. The polymer may be linear, cyclic, or branched, and it may comprise modified amino acids, particularly conservatively modified amino acids, and it may be interrupted by non-amino acids. The term also includes modified amino acid polymers such as amino acid polymers that have been modified by sulfation, glycosylation, lipidation, acetylation, phosphorylation, iodination, methylation, oxidation, proteolytic processing, prenylation, racemization, selenoylation, transfer RNA-mediated amino addition such as arginylation, ubiquitination, or any other manipulation such as conjugation with a labeling component. As used herein, the term "amino acid" refers to natural and/or non-natural or synthetic amino acids, including glycine and D or L optical isomers, as well as amino acid analogs and peptidomimetics. A polypeptide or amino acid sequence "derived from" a specified protein refers to the source of the polypeptide. The term also includes polypeptides expressed by a specified nucleic acid sequence.

The term "amino acid modification" (or "modified amino acid") includes amino acid substitutions, insertions, and/or deletions in a polypeptide sequence. As used herein, "amino acid substitution", "substitution", or "replacement" refers to the replacement of an amino acid at a specific position in a parent polypeptide sequence with another amino acid. For example, S32A substitution refers to the replacement of serine at position 32 with alanine.

"Specifically binding" means that the binding is selective for the antigen and may be distinguished from unwanted or non-specific interactions. The ability of an antibody to bind to a specific antigenic determinant may be determined by enzyme-linked immunosorbent assay (ELISA) or other techniques familiar to those skilled in the art, such as surface plasmon resonance (SPR) technique (analyzed on a BIAcore instrument) (Liljeblad et al., Glyco J, 17, 323-329 (2000)) and traditional binding assays (Heeley Endocr, Res, 28, 217-229 (2002)).

"Affinity" refers to the strength of the sum of non-covalent interactions between a single binding site of a molecule (*e.g.,* a receptor) and its binding partner (*e.g.,* a ligand). Unless otherwise indicated, "binding affinity" as used herein refers to intrinsic binding affinity, which reflects a 1:1 interaction between members of a binding pair (*e.g.,* an antigen-binding portion and an antigen, or a receptor and its ligand). The affinity of a molecule X for its partner Y may generally be represented by the dissociation constant (K_{D}), which is the ratio of the dissociation and association rate constants (k_{off} and kₒₙ, respectively). Thus, equivalent affinities may encompass different rate constants, provided the ratio of the rate constants remains the same. Affinity may be measured by well-established methods known in the art, including those described herein. The specific method for measuring affinity is surface plasmon resonance (SPR).

As used herein, the term "valence" refers to the presence of a specified number of antigen-binding regions in an antigen-binding molecule.

An "antigen-binding site" refers to the site of an antigen-binding molecule, *i.e.,* one or more amino acid residues, that provides interaction with an antigen. For example, the antigen-binding site of an antibody includes amino acid residues from the complementarity-determining regions (CDRs).

The term "antibody" is used herein in its broadest sense to encompass a variety of antibody structures including, but not limited to, monoclonal antibodies, polyclonal antibodies, and antibody fragments, as long as they exhibit the desired antigen-binding activity.

As used herein, the term "GPC3", also known as Glypican-3, is a proteoglycan anchored to the cell surface via glycosylphosphatidylinositol (GPI). It belongs to the heparan sulfate proteoglycan (HSPG) family and is primarily involved in regulating cell proliferation, adhesion, and migration. The GPC3 gene is located on human chromosome X (Xq26.1) and encodes a GPC3 protein precursor containing 580 amino acid residues (Nat Genet, 1996, 12(3): 241-7). The GPC3 core protein is approximately 70 kDa and contains a unique sequence of 14 cysteine residues (Cys). Furin protease cleaves the core protein (cleavage site between Arg358 and Cys359) to produce a soluble N-terminal peptide of approximately 40 kDa and a membrane-bound C-terminal peptide of approximately 30 kDa containing two heparan sulfate (HS) glycan chains (Eur J Cancer, 2011, 47(3): 333-8; Proc Natl Acad Sci USA, 2011, 108(32): 13112-7).

As used herein, the terms "anti-GPC3 antibody", "anti-GPC3 nanobody", "antibody against GPC3", and "nanobody against GPC3" refer to an antibody that is capable of binding to a GPC3 protein or a fragment thereof with sufficient affinity, such that the antibody may be used as a diagnostic and/or therapeutic agent targeting GPC3. The GPC3 protein derived from mouse is denoted as mGPC3, the GPC3 protein derived from cynomolgus macaque is denoted as cyno GPC3, and the GPC3 protein derived from human is denoted as huGPC3. The terms "anti-human GPC3 antibody", "anti-human GPC3 nanobody", "anti-huGPC3 antibody", "anti-huGPC3 nanobody", "antibody against huGPC3", and "nanobody against huGPC3" specifically refer to an antibody that is capable of binding to a human GPC3 protein or a fragment thereof with sufficient affinity, such that the antibody may be used as a diagnostic and/or therapeutic agent targeting human GPC3.

As used herein, the term "anti-CD3 antibody" refers to an antibody that can specifically bind to an individual CD3 chain (*e.g.,* a CD3 (γ) chain, a CD3 (δ) chain, or a CD3 (ε) chain) or a complex formed by two or more individual CD3 chains (*e.g.,* a complex of more than one CD3 (ε) chain, a complex of a CD3 (γ) chain and a CD3 (ε) chain, or a complex of a CD3 (δ) chain and a CD3 (ε) chain). In certain embodiments, the anti-CD3 antibody can specifically bind to CD3 (γ), CD3 (δ), or CD3 (ε), or any combination thereof; more preferably, the anti-CD3 antibody can specifically bind to CD3 (ε). The terms "anti-human CD3 antibody" and "anti-hCD3 antibody" refer to an antibody that can specifically bind to human-derived CD3.

It should be noted that the divisions of CDR and FR in the variable regions of the antibody of the present disclosure are determined according to the Kabat definition. Other nomenclature and numbering systems, such as Chothia, IMGT, or AHo, are also known to those skilled in the art. Therefore, humanized antibodies comprising one or more CDRs derived from any nomenclature system, based on the antibody sequences of the present disclosure, are explicitly encompassed within the scope of the present disclosure.

The term "sequence identity", "sequence similarity", or "sequence homology" refers to the percentage of amino acid residues in a candidate sequence that are identical to those in a reference polypeptide sequence after aligning the sequences (and introducing gaps if necessary) to achieve the maximum percent sequence identity, without considering any conservative substitutions as part of the sequence identity. Sequence alignments may be performed to determine percent amino acid sequence identity using various methods in the art, for example, using publicly available computer software such as BLAST, BLAST-2, ALIGN, or MEGALIGN (DNASTAR) software. Those skilled in the art may determine appropriate parameters for measuring alignment, including any algorithms required to achieve maximum alignment over the full length of the sequence being compared.

Conventional immunoglobulins are tetramers consisting of two heavy chains and two light chains, with a combined molecular weight of approximately 150 kDa. In members of the Camelidae family, a significant proportion of serum antibodies are homodimeric IgGs with a molecular weight of approximately 80 kD (Hamers-Casterman et al., 1993, Nature, 363, 446-448). These heavy-chain immunoglobulins (Ig) contain three domains, and their variable region is referred to as VHH (variable domain of heavy chain of heavy-chain antibody). Recombinant VHHs (approximately 12 to 14 kD) constitute intact antigen-binding domains and exhibit a broad antigen-binding repertoire. Their hypervariable regions are expanded and exhibit unique characteristics, such as the substitution of three to four hydrophobic framework residues (which interact with the VL in conventional antibodies) by more hydrophilic amino acids. To stabilize the expanded CDRs, VHHs may have additional disulfide bonds between CDR1 and CDR3 in dromedaries and between CDR2 and CDR3 in llamas, in addition to the conventional disulfide bonds (Harmsen and De Haard, 2007, Appl Microbiol Biotechnol., 77, 13-22; Muyldermans, 2001, J Biotechnol., 74, 277-302). The expanded CDR3 loop may adopt a convex conformation, whereas conventional paratopes are limited to concave or flat structures (Muyldermans, 2001, J Biotechnol., 74, 277-302). These features allow VHHs to recognize unique epitopes that are poorly immunogenic for conventional antibodies (Lafaye, 2009, Mol Immunol., 46, 695-704; Wernery, 2001, J Vet Med B Infect Dis Vet Public Health., 48, 561-568). Although VHHs are defined as monovalent antibodies, which by default exclude any avidity effects, their biological activity measured as IC₅₀ *in vitro* may be similar to that of conventional bivalent antibody molecules (Thys et al., 2010, Antiviral Res., 87, 257-264).

In certain embodiments, the present disclosure may relate to chimeric camelid/human antibodies, particularly chimeric antibodies in which the VH and/or VL domains are of fully camelid sequence (*e.g.,* llama or alpaca), while the remainder of the antibody is of fully human sequence. In some preferred embodiments of the present disclosure, further included are "humanized" or "germlined" camelid antibodies and camelid/human chimeric antibodies, in which the VH and/or VL domains contain one or more amino acid substitutions in the framework regions compared to the camelid VH and/or VL domains obtained by active immunization. The "humanization" process involves replacing mismatched amino acid residues in a starting camelid VH or VL domain with corresponding residues in a human germline VH or VL domain, thereby increasing the percent sequence identity with the human germline VH or VL domain.

The present disclosure includes natural and recombinant VHH or VH.

According to the present disclosure, the VHH can be in the form of a monomer or in the form of a homomultimer, such as a homodimer or a homotrimer.

The antibodies of the present disclosure include camelid antibodies, chimeric antibodies, and humanized antibodies, preferably humanized antibodies.

The term "chimeric antibody" refers to a construct in which a portion of the heavy and/or light chain is identical or homologous to a corresponding sequence in an antibody from a particular species or belonging to a particular antibody class or subclass, while the remaining portion of the chain(s) is identical or homologous to a corresponding sequence in an antibody from another species or belonging to another antibody class or subclass, as well as corresponding sequences in fragments of such antibodies. In a narrow sense, a chimeric antibody comprises all or most of selected murine heavy and light chain variable regions operably linked to human light and heavy chain constant regions. The constant region sequences, or variants or derivatives thereof, may be operatively associated with the disclosed heavy and light chain variable regions using standard molecular biology techniques to provide full-length anti-GPC3 antibodies that may be used per se or may be incorporated into the present disclosure.

The term "humanized antibody" refers to a hybrid immunoglobulin, immunoglobulin chain or fragment thereof containing a minimal sequence derived from a non-human immunoglobulin. In most cases, the humanized antibody is a human immunoglobulin (receptor antibody) in which residues from CDRs of the receptor are replaced with residues from CDRs of a non-human species (donor antibody) having the desired specificity, affinity and properties, such as mouse, rat, rabbit, or primate. In some cases, framework region residues of a human immunoglobulin are replaced with corresponding non-human residues. In certain cases, "back mutations" may be introduced into a humanized antibody in which residues in one or more FRs of the variable region of a human receptor antibody are replaced with corresponding residues from a non-human donor antibody. Such back mutations may help maintain the proper three-dimensional configuration of one or more grafted CDRs and thus improve affinity and antibody stability. Antibodies from a variety of donor species including, but not limited to, mice, rats, rabbits, or non-human primates may be used. In addition, the humanized antibody may contain new residues not found in the receptor antibody or in the donor antibody to further improve antibody performance.

The term "single domain antibody", also known as nanobody, may be defined as an amino acid sequence with the following (general) structure: FR1-CDR1-FR2-CDR2-FR3-CDR3-FR4, wherein FR1 to FR4 refer to framework regions (Frame) 1 to 4, respectively, and wherein CDR1 to CDR3 refer to complementarity-determining regions 1 to 3, respectively. "VHH" refers to variable antigen-binding domains of heavy chain antibodies from camelids (camels, dromedaries, llamas, alpacas, *etc*.) (see Nguyen, 2000, EMBO J., 19, 921-930; Muyldermans, 2001, J Biotechnol., 74, 277-302; and reviewed by Vanlandschoot, 2011, Antiviral Res., 92, 389-407).

The term "scFv" refers to a fusion protein comprising at least one antibody fragment comprising a light chain variable region and at least one antibody fragment comprising a heavy chain variable region, wherein the light chain and heavy chain variable regions are contiguous (*e.g.,* via a synthetic linker such as a short flexible polypeptide linker) and can be expressed as a single-chain polypeptide, and wherein the scFv retains the specificity of the intact antibody from which it is derived. Unless otherwise specified, the scFv may have the VL and VH variable regions in any order (*e.g.,* relative to the N-terminus and C-terminus of the polypeptide), and the scFv may comprise VL-linker-VH or may comprise VH-linker-VL.

As known in the art, an antibody is divided into five major classes: IgA, IgD, IgE, IgG, and IgM, with the corresponding heavy chain constant domains called α, δ, ε, γ, and µ, respectively. IgG and IgA may be further divided into different subclasses. For example, IgG may be subdivided into IgG1, IgG2, IgG3, and IgG4, and IgA may be subdivided into IgA1 and IgA2. The light chains of antibodies from any vertebrate species may be assigned to one of two distinct types, called κ and λ, based on the amino acid sequences of their constant domains.

In IgA, IgG, and IgD, the constant region comprises three domains called CH1, CH2, and CH3 (IgM and IgE have a fourth domain, CH4). In IgG, IgA, and IgD, the CH1 and CH2 domains are separated by a flexible hinge region, which is a proline- and cysteine-rich segment of variable length. Each class of antibodies further comprises interchain and intrachain disulfide bonds formed by paired cysteine residues.

The term "Fc" is used herein to define the C-terminal region of an immunoglobulin heavy chain, *i.e.,* the two polypeptide chains forming a dimer comprising the C-terminal constant region of the immunoglobulin heavy chain that is capable of stabilizing self-association. The term includes native sequence Fc regions and variant Fc regions. Although the boundaries of the Fc region of an IgG heavy chain may vary slightly, the Fc region of a human IgG heavy chain is typically defined as extending from Cys226 or Pro230 to the C-terminus of the heavy chain. For example, the IgG Fc domain comprises the IgG CH2 and IgG CH3 constant domains. Unless otherwise specified herein, numbering of amino acid residues in the Fc region or constant region follows the EU numbering system, also referred to as the EU index, as described in Kabat et al., Sequences of Proteins of Immunological Interest, 5th Ed., Public Health Service, National Institutes of Health, Bethesda, MD, 1991.

A "modification promoting association of the first and second subunits of the Fc domain" refers to a manipulation of the peptide backbone or the post-translational modifications of an Fc domain subunit that reduces or prevents the association of a polypeptide comprising the Fc domain subunit with an identical polypeptide to form a homodimer. As used herein, the modification promoting association particularly includes separate modifications made to each of the two Fc domain subunits desired to associate (*i.e.,* the first and second subunits of the Fc domain), wherein the modifications are complementary to each other so as to promote association of the two Fc domain subunits. For example, the modification promoting association may alter the structure or charge of one or both of the Fc domain subunits so as to make their association sterically or electrostatically favorable, respectively. Thus, (hetero)dimerization occurs between a polypeptide comprising the first Fc domain subunit and a polypeptide comprising the second Fc domain subunit, which may be non-identical in the sense that the other components fused to each of the subunits (*e.g.,* Fab fragments) are not the same. In some embodiments, the modification promoting association comprises an amino acid mutation, specifically an amino acid substitution, in the Fc domain. In a specific embodiment, the modification promoting association comprises a separate amino acid mutation, specifically an amino acid substitution, in each of the two subunits of the Fc domain.

There are several approaches to modify the CH3 domain of the Fc domain to enhance heterodimerization, which are described in detail in, for example, WO96/27011, WO98/050431, EP1870459, WO2007/110205, WO2007/147901, WO2009/089004, WO2010/129304, WO2011/90754, WO2011/143545, WO2012/058768, WO2013/157954, and WO2013/096291. Typically, in all such approaches, both the CH3 domain of the first polypeptide chain of the Fc domain and the CH3 domain of the second polypeptide chain of the Fc domain are engineered in a complementary manner such that each CH3 domain (or the heavy chain comprising CH3 domain) is no longer able to homodimerize with itself but is forced to heterodimerize with other complementarily engineered CH3 domains (such that the first and second CH3 domains are heterodimerized and no homodimer is formed between the two first CH3 domains or the two second CH3 domains).

In a particular embodiment, the modification promoting association of the first polypeptide chain and the second polypeptide chain of the Fc domain is a so-called "Knob-into-Hole" modification, comprising a "Knob" modification in one of the two polypeptide chains of the Fc domain and a "Hole" modification in the other of the two polypeptide chains of the Fc domain.

Knob-into-Hole techniques are described in, for example, US5731168; US7695936; Ridgway et al., Prot Eng, 9, 617-621 (1996) and Carter, J Immunol Meth, 248, 7-15 (2001). Generally, the method involves introducing a protuberance ("Knob") at the interface of the first polypeptide chain and a corresponding cavity ("Hole") at the interface of the second polypeptide chain, such that the protuberance may be placed in the cavity to promote heterodimer formation and hinder homodimer formation. The protuberance is constructed by replacing a small amino acid side chain from the first polypeptide chain interface with a larger side chain (*e.g.,* tyrosine or tryptophan). A complementary cavity having the same or similar size as the protuberance is created at the interface of the second polypeptide chain by replacing the large amino acid side chain with a smaller amino acid side chain (*e.g.,* alanine or threonine).

Thus, in a specific embodiment, in the CH3 domain of the first polypeptide chain of the Fc domain of the bispecific antigen-binding molecule of the present disclosure, one amino acid residue is replaced with an amino acid residue having a larger side chain volume, thereby generating a protuberance in the CH3 domain of the first polypeptide chain, which may be placed in a cavity in the CH3 domain of the second polypeptide chain, and in the CH3 domain of the second polypeptide chain of the Fc domain, one amino acid residue is replaced with an amino acid residue having a smaller side chain volume, thereby generating a cavity in the CH3 domain of the second polypeptide chain, in which the protuberance in the CH3 domain of the first polypeptide chain may be placed.

Preferably, the amino acid residue having a larger side chain volume is selected from the group consisting of arginine (R), phenylalanine (F), tyrosine (Y), and tryptophan (W). Preferably, the amino acid residue having a smaller side chain volume is selected from the group consisting of alanine (A), serine (S), threonine (T), and valine (V).

Protuberances and cavities may be generated by altering the nucleic acid encoding the polypeptide, for example, by site-specific mutagenesis or by peptide synthesis.

As used herein, "Knob-Fc" refers to a point mutation, T366W, comprised in the Fc region of an antibody to form a Knob-like spatial structure. Correspondingly, "Hole-Fc" refers to point mutations, T366S, L368A, and Y407V, comprised in the Fc region of an antibody to form a Hole-like spatial structure. Knob-Fc and Hole-Fc are more likely to form heterodimers due to steric hindrance. To reduce the production of hole-hole homodimers, H435R mutation is introduced into Hole, which may reduce the binding to protein A during purification. In order to further promote the formation of heterodimers, point mutations S354C and Y349C may also be introduced into Knob-Fc and Hole-Fc, respectively, to further promote the formation of heterodimers through disulfide bonds. Meanwhile, in order to eliminate or weaken the ADCC effect caused by antibody Fc, substitution mutations L234A and L235Amay also be introduced into Fc. For example, Knob-Fc and Hole-Fc of the present disclosure are preferably as shown in SEQ ID NOs: 14 and 15, respectively. In a bispecific antigen-binding molecule, Knob-Fc or Hole-Fc may be used as either the Fc region of the first polypeptide chain or the Fc region of the second polypeptide chain, and in the same bispecific antigen-binding molecule, the Fc region of the first polypeptide chain and the Fc region of the second polypeptide chain are not both Knob-Fc or Hole-Fc.

It should be noted that the divisions of CDR and FR in the variable regions of the monoclonal antibody of the present disclosure are determined according to the Kabat definition. Other nomenclature and numbering systems, such as Chothia, IMGT, or AHo, are also known to those skilled in the art. Therefore, humanized antibodies comprising one or more CDRs derived from any nomenclature system, based on the monoclonal antibody sequences of the present disclosure, are explicitly encompassed within the scope of the present disclosure.

As used herein, "homologous" and "heterologous" are a set of relative concepts, which may refer to different elements in a construct having the same or different sources, or may refer to the fact that after the construct has been constructed, among multiple elements that are originally derived from the same source, *i.e.,* "homologous", some of the elements have been engineered and changed from other original elements that have not been engineered, thereby becoming "heterologous".

The term "fusion" means that the components (*e.g.,* scFv antibody and Fc domain subunit) are linked by a peptide bond, either directly or via one or more peptide linkers.

The term "linker" refers to any means for linking two different functional units (*e.g.,* antigen-binding fragments). Types of linkers include, but are not limited to, chemical linkers and polypeptide linkers. The sequence of the polypeptide linker ("peptide linker") is not limited. Polypeptide linkers are preferably non-immunogenic and flexible, such as those comprising serine and glycine sequences. Depending on the specific construct, the linker may be long or short.

The term "antibody-dependent cell-mediated cytotoxicity" (ADCC) refers to the binding of an antibody to an antigenic epitope of a virus-infected cell or tumor cell, wherein Fc fragments bind to Fc receptors (FcRs) on the surface of killer cells (NK cells, macrophages, *etc*.) to mediate the direct killing of target cells by killer cells.

As used herein, the term "engineering" includes any manipulation of the peptide backbone or the post-translational modifications of a naturally occurring or recombinant polypeptide or fragment thereof. Engineering includes modifications of amino acid sequences, glycosylation patterns, or side chain groups of individual amino acids, as well as combinations of these approaches.

The term "pharmaceutical composition" refers to a preparation in a form that allows the biological activity of the active ingredient contained therein to be effective, and which does not contain additional ingredients having unacceptable toxicity to a subject to whom the preparation is administered.

The term "pharmaceutical carrier" or "pharmaceutically acceptable carrier" refers to a diluent, adjuvant (*e.g.,* Freund's adjuvant (complete and incomplete)), excipient, or vehicle with which a therapeutic agent is administered.

The term "effective amount" refers to a dose of a pharmaceutical preparation comprising the active ingredient of the present disclosure that, when administered to a patient in a single or multiple doses, produces the desired effect in the treated patient. The effective amount may be readily determined by the attending physician, as one skilled in the art, by taking into account a variety of factors, such as racial differences; body weight, age, and health; the specific disease involved; the severity of the disease; the response of the individual patient; the specific antibody administered; the mode of administration; bioavailability characteristics of the preparation administered; the dosing regimen selected; and the use of any concomitant therapy.

The terms "host cell", "host cell line", and "host cell culture" are used interchangeably to refer to a cell into which an exogenous nucleic acid has been introduced, including the progeny of such a cell. Host cells include "transformants" and "transformed cells", which include primary transformed cells and progeny derived therefrom, regardless of the number of passages. The progeny may not be identical to a parental cell in nucleic acid content, but may contain mutations. Mutant progeny that have the same function or biological activity as screened or selected for in the originally transformed cell are included herein. A host cell is any type of cellular system that may be used to produce the bispecific antigen-binding molecule of the present disclosure. Host cells include cultured cells, for example, mammalian cultured cells, such as Jurkat cells, PBMCs, HepG2 cells, Hep3B cells and Huh-7 cells or hybridoma cells, yeast cells, insect cells, and plant cells, as well as cells contained in transgenic animals, transgenic plants, or cultured plant or animal tissues.

As used herein, the term "transfection" refers to the introduction of an exogenous nucleic acid into a eukaryotic cell. Transfection may be accomplished by a variety of means known in the art, including calcium phosphate-DNA co-precipitation, DEAE-dextran-mediated transfection, polybrene-mediated transfection, electroporation, microinjection, liposome fusion, lipid transfection, protoplast fusion, retroviral infection, and biolistics.

The term "stable transfection" or "ST" refers to the introduction and integration of an exogenous nucleic acid, DNA, or RNA into the genome of a transfected cell. The term "stable transfectant" refers to a cell that stably integrates foreign DNA into genomic DNA.

The term "isolated polynucleotide" refers to a nucleic acid molecule, DNA, or RNA, which has been removed from its natural environment. For example, a recombinant polynucleotide encoding a polypeptide contained in a vector is considered isolated for the purposes of the present disclosure. Further examples of isolated polynucleotides include recombinant polynucleotides maintained in heterologous host cells or (partially or substantially) purified polynucleotides in solution. The isolated polynucleotide includes a polynucleotide molecule contained in a cell that ordinarily contains the polynucleotide molecule, but the polynucleotide molecule is present extrachromosomally or at a chromosomal location that is different from its natural chromosomal location. Isolated RNA molecules include *in vivo* or *in vitro* RNA transcripts of the present disclosure, as well as positive and negative stranded forms and double stranded forms. The isolated polynucleotides or nucleic acids of the present disclosure further include such molecules produced synthetically. In addition, the polynucleotide or nucleic acid may or may not include a regulatory element, such as a promoter, a ribosome binding site, or a transcription terminator.

Methods for producing and purifying antibodies and antigen-binding fragments are well known and can be found in the art, for example, in Antibodies: A Laboratory Manual, Cold Spring Harbor Laboratory Press, Chapters 5 to 8 and 15. The antibodies or antigen-binding fragments thereof of the present disclosure are genetically engineered to add one or more human FR regions to the non-human CDR region. Human FR germline sequences are available from the website (http://imgt.cines.fr) of ImMunoGeneTics (IMGT), or from The Immunoglobulin FactsBook, (2001) ISBN: 012441351.

The engineered antibodies or antigen-binding fragments thereof of the present disclosure may be prepared and purified by conventional methods. For example, cDNA sequences encoding heavy and light chains may be cloned and recombined into expression vectors. Recombinant immunoglobulin expression vectors may be stably transfected into CHO cells. As a more recommended prior art, mammalian expression systems result in glycosylation of antibodies, particularly at the highly conserved N-terminus of the Fc region. Stable clones are obtained by expressing antibodies that specifically bind to human antigens. Positive clones are expanded in serum-free medium in a bioreactor for antibody production. The culture medium with secreted antibodies may be purified and collected using conventional techniques. Antibodies may be concentrated by filtration using conventional methods. Soluble mixtures and polymers may also be removed by conventional methods, such as molecular sieves and ion exchange.

As used herein, the term "individual" or "subject" refers to any animal, such as a mammal or a marsupial. Individuals of the present disclosure include, but are not limited to, humans, non-human primates (*e.g.,* cynomolgus macaques or rhesus macaques or other types of macaques), mice, pigs, horses, donkeys, cattle, sheep, rats, and any kind of poultry.

As used herein, the term "disease", "condition", or "disorder" refers to any alteration or disorder that impairs or interferes with the normal function of a cell, tissue, or organ. For example, the "disease" includes, but is not limited to, tumors, pathogen infections, autoimmune diseases, T cell dysfunctions, or deficiencies in immune tolerance (*e.g.,* transplant rejection).

As used herein, the term "treatment" refers to clinical intervention in an attempt to alter an individual or treat a cell-induced disease, either prophylactically or in a clinical pathological process. Therapeutic effects include, but are not limited to, prevention of the onset or recurrence of a disease, alleviation of symptoms, reduction of any direct or indirect pathological consequences of a disease, prevention of metastasis, slowing of the rate of progression of a disease, amelioration or remission of a condition, remission or amelioration of a prognosis, *etc.*

### DETAILED DESCRIPTION OF THE PREFERRED EMBODIMENT

The present disclosure will be described in detail below in conjunction with specific examples. It should be understood that these examples are only used to describe the present disclosure and are not intended to limit the scope of the present disclosure. The experimental methods for which specific conditions are not indicated in the following examples are typically carried out according to conventional conditions such as those described in Molecular Cloning: A Laboratory Manual (Third Edition) by J. Sambrook et al., Science Press, 2002, or according to the conditions recommended by the manufacturer.

### DNA sequencing

DNA sequences are determined by duplex sequencing.

### Preparation and screening of antibodies

Methods for preparing monoclonal antibodies are known in the art. One method that may be used is the method as described in Kohler G. et al., "Continuous Cultures of Fused Cells Secreting Antibody of Predefined Specificity", Nature, 256: 495-497 (1975) or a modified form thereof.

### Antigen-binding domain

For example, the light chain variable region (VL) and heavy chain variable region (VH) of the CD3 antigen-binding domain scFv are derived from the sequences disclosed in WO2021022304A2. Exemplary anti-CD3 CDR, VH, VL, or scFv sequences are shown in Table 1.

**Table 1: Sequence list of CD3 antigen-binding domains in bispecific antigen-binding molecules**

| Name | SEQ ID NO: | Sequence |
|---|---|---|
| VH_{CD3} | 1 | EVKLVESGGGLVQPGGSLKLSCAASGFTFNTYAMNWVRQAPGKGLE WVARIRSKYNNYATYYADSVKDRFTISRDDAKNTLYLQMNNLRTED TAVYYCVRHGNFGNSYVSWFAYWGQGTLVTVSS |
| VL_{CD3} | 2 | EVKLVESGGGLVQPGGSLKLSCAASGFTFNTYAMNWVRQAPGKGLE WVARIRSKYNNYATYYADSVKDRFTISRDDAKNTLYLQMNNLRTED TAVYYCVRHGNFGNSYVSWFAYWGQGTLVTVSS QAVVTQEPSLTVSPGGTVTLTCRSSTGAVTTSNYANWVQQKPGQAPR GLIGGTNKRAPGTPARFSGSLLGGKAALTLSGAQPEDEAEYYCALW YSNLWVFGGGTKLTVL EVKLVESGGGLVQPGGSLKLSCAASGFTFNTYAMNWVRQAPGKGLE WVARIRSKYNNYATYYADSVKDRFTISRDDAKNTLYLQMNNLRTED TAVYYCVRHGNFGNSYVSWFAYWGQGTLVTVSSGGGGSGGGGSG GGGSAQAVVTQEPSLTVSPGGTVTLTCRSSTGAVTTSNYANWVQQK PGQAPRGLIGGTNKRAPGTPARFSGSLLGGKAALTLSGAQPEDEAEY YCALWYSNLWVFGGGTKLTVL |
| scFv_{CD3} | 3 | EVKLVESGGGLVQPGGSLKLSCAASGFTFNTYAMNWVRQAPGKGLE WVARIRSKYNNYATYYADSVKDRFTISRDDAKNTLYLQMNNLRTED TAVYYCVRHGNFGNSYVSWFAYWGQGTLVTVSSGGGGSGGGGSG GGGSAQAVVTQEPSLTVSPGGTVTLTCRSSTGAVTTSNYANWVQQK PGQAPRGLIGGTNKRAPGTPARFSGSLLGGKAALTLSGAQPEDEAEY YCALWYSNLWVFGGGTKLTVL |
| HCDR1_{CD3} | 4 | TYAMN |
| HCDR2_{CD3} | 5 | RIRSKYNNYATYYADSVKD |
| HCDR3_{CD3} | 6 | HGNFGNSYVSWFAY |
| LCDR1_{CD3} | 7 | RSSTGAVTTSNYAN |
| LCDR2_{CD3} | 8 | GTNKRAP |
| LCDR3_{CD3} | 9 | ALWYSNLWV |

Exemplary GPC3 antigen-binding domain sequences are shown in Table 2.

**Table 2: Sequence list of GPC3 antigen-binding domains in bispecific antigen-binding molecules**

| Name | SEQ ID NO: | Sequence |
|---|---|---|
| VHH_{GPC3} | 10 | QVQLVESGGGVVQPGGSLRLSCAASGRTLTNYVMGWFRQAP GKEREIVAGINWAGSRIGYGDFVKGRFTISRDNSKNTVYLQM NSLRPEDTAVYYCAAKLGKYNYPEYDYRGQGTLVTVSS |
| VHH_{GPC3}-HCDR-1 | 11 | NYVMG |
| VHH_{GPC3}-HCDR-2 | 12 | GINWAGSRIGYGDFVKG |
| VHH_{GPC3}-HCDR-3 | 13 | KLGKYNYPEYDY |

### Molecular structure (Format) of bispecific antigen-binding molecules

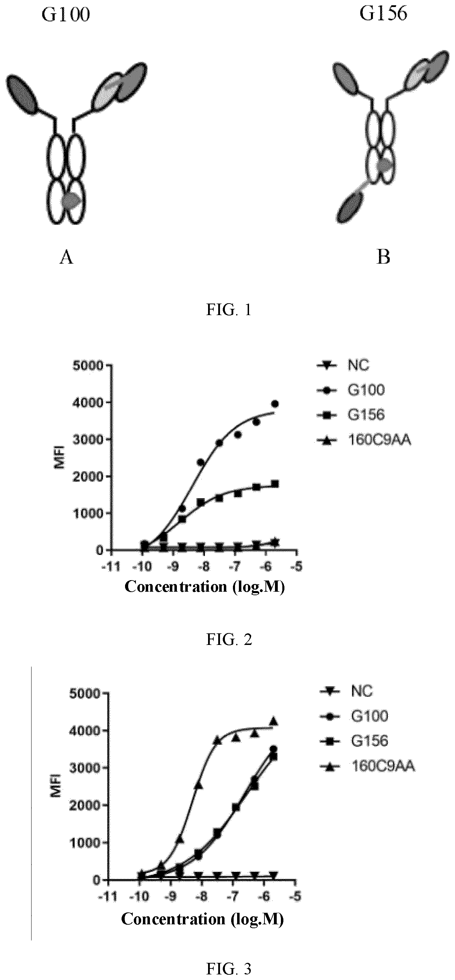

In some embodiments disclosed herein, the bispecific antigen-binding molecule has a structure consisting of two peptide chains, as shown in FIG. 1. Exemplarily, when nanobody VHH targets GPC3 and scFv antibody targets CD3, two optimized molecular structures (Formats) of bispecific antigen-binding molecules are obtained through screening for the above targets, as shown in Table 3 below, in which Format 1 and Format 2 correspond to A in FIG. 1 and B in FIG. 1, respectively.

**Table 3: Molecular structure of bispecific antigen-binding molecules**

| Name of molecular structure | Arrangement of the first polypeptide chain | Arrangement of the second polypeptide chain |
|---|---|---|
| Format 1 | VHH_{GPC3}-Linker-Knob-Fc | VH_{CD3}-Linker-VL_{CD3}-Linker-Hole-Fc |
| Format 2 | VHH_{GPC3}-Linker-Knob-Fc-Linker-VHH_{GPC3} | VH_{CD3}-Linker-VL_{CD3}-Linker-Hole-Fc |

| | | |
|---|---|---|
| Note: Linker indicates a peptide linker, which is used to link antigen-binding domains and the Fc region. | | |

### Peptide linker sequences of bispecific antigen-binding molecules

The peptide linker used to link antigen-binding domains and the Fc region may be selected from any other peptide linker that may be used to link functional domains of an antibody, and is not limited to the peptide linker limited by the following sequence. Exemplarily, the peptide linker may be G₄S, (G₄S)₂, (G₄S)₃, (G₄S)₄, (G₄S)₁A, (G₄S)₂A, (G₄S)₃A, (G₄S)₄A, EPKSSDKTHTCPPCP, or DKTHTCPPCP.

### Fc sequence

As shown in Table 3, the bispecific antigen-binding molecule may further include an Fc region (*i.e.,* Fc domain). The Fc region may be Knob-Fc or Hole-Fc, and can maintain the normal half-life and good stability of the antibody. In a preferred embodiment, the design of two chains achieves the purpose of greatly reducing the probability of mismatch, which may further improve the homogeneity of the sample and the yield of the target antibody. Exemplarily, the bispecific antigen-binding molecule is a bispecific heterodimer consisting of a Knob-Fc chain and a Hole-Fc chain, wherein the Knob-Fc chain has a structure comprising amino acid substitutions at two sites, S354C and T366W, and the Hole-Fc chain has a structure comprising amino acid substitutions at four sites, Y349C, T366S, L368A, and Y407V. Moreover, to facilitate purification of the bispecific antigen-binding molecule, the Hole-Fc chain may further comprise an H435R substitution. In addition, to reduce the ADCC activity of the antibody, the Fc fragment may further comprise L234A and L235A amino acid substitutions. For example, as shown in Table 4, the sequence of the engineered Knob-Fc chain is as shown in SEQ ID NO: 14, and the sequence of the engineered Hole-Fc chain is as shown in SEQ ID NO: 15. In addition, to prevent breakage between the variable region of the anti-human GPC3 nanobody and the C-terminus of the heavy chain, a K447A amino acid substitution, *i.e.,* a K to A mutation, may be optionally introduced at the last position of the C-terminus of the heavy chain.

**Table 4: Sequence list of different Fc chains**

| Name | SEQ ID NO: | Sequence |
|---|---|---|
| Knob-Fc | 14 | APEAAGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPCRDELTKNQVSLWCLV KGFYPSDIAVEWESNGQPENNYKTTPPVLDSDGSFFLYSKLTVDKSR WQQGNVFSCSVMHEALHNHYTQKSLSLSPGK |
| Hole-Fc | 15 | APEAAGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVCTLPPSRDELTKNQVSLSCAVK GFYPSDIAVEWESNGQPENNYKTTPPVLDSDGSFFLVSKLTVDKSRW QQGNVFSCSVMHEALHNRYTQKSLSLSPGK |

### Examples

### Example 1: Preparation, expression, and purification of bispecific antigen-binding molecules

The bispecific antigen-binding molecule G100 adopted the Format-1 molecular structure, with the specific sequences as shown in Table 5. The bispecific antigen-binding molecule G156 adopted the Format-2 molecular structure, with the specific sequences as shown in Table 6.

The DNA sequences encoding the amino acids of the aforementioned bispecific antigen-binding molecules G100 and G156 were cloned into prokaryotic or eukaryotic expression vectors, respectively. The expression vectors were then introduced into prokaryotic or eukaryotic hosts for the expression of the polypeptide or antigen-binding fragment. After sequencing, completely correct clones were selected for transfection and expression, as well as cell culture. After 8 to 10 days of cell culture, the expression supernatant was collected, centrifuged at high speed to remove cell debris, followed by affinity purification using a Protein A column. The column was rinsed with PBS until the A280 reading was decreased to baseline. The target protein was eluted with an acidic elution buffer at pH 3.0 to 3.5 and neutralized with 1 M Tris-HCl at pH 8.0 to 9.0. The eluted samples were properly concentrated, buffer exchanged into PBS, and aliquoted for storage. The final purified humanized antibody was subjected to SDS-PAGE and HPLC purity analysis and A280 concentration measurement.

**Table 5: Sequence list of bispecific antigen-binding molecule G100**

| Antibody name | The first polypeptide chain (structure: Format-1): VHH_{GPC3}-Linker-Knob-Fc | The second polypeptide chain (structure: Format-1): VH_{CD3}-Linker-VL_{CD3}-Linker-Hole-Fc |
|---|---|---|
| G100 | SEQ ID NO: 16 | SEQ ID NO: 17 |

**Table 6: Sequence list of bispecific antigen-binding molecule G156**

| Antibody name | The first polypeptide chain (structure: Format-1): VHH_{GPC3}-Linker-Knob-Fc-Linker-VHH_{GPC3} | The second polypeptide chain (structure: Format-1): VH_{CD3}-Linker-VL_{CD3}-Linker-Hole-Fc |
|---|---|---|
| G156 | SEQ ID NO: 18 | SEQ ID NO: 17 |

### Example 2: Construction of anti-CD3-GPC3 bispecific antibody and transient transfection expression in eukaryotic cells

The target gene fragments of the aforementioned bispecific antibody and anti-CD3 positive monoclonal antibody control molecule were cloned into PTT5 expression vectors, respectively, to prepare transfection-grade expression plasmids. The plasmids were inoculated into HEK293E or Expi293 cells cultured in serum-free medium and incubated on a shaker at 37°C with 8% CO₂. After 6 days of cell culture, the supernatant was collected for purification. The final purified bispecific antibody was subjected to SDS-PAGE and HPLC purity analysis and A280 concentration measurement.

### Example 3: Affinity detection assay of anti-CD3-GPC3 bispecific antibody

A. Affinity detection of anti-CD3-GPC3 bispecific antibody for cells expressing human GPC3 and human CD3

The binding of anti-CD3-GPC3 bispecific antibody to human hepatocellular carcinoma HepG2 cells (ATCC, Cat. No.: HB-8065) naturally expressing human GPC3 and Jurkat cells (ATCC, Cat. No.: TIB-152) naturally expressing human CD3 was detected using FACS.

The above cells were collected separately, resuspended in FACS buffer (PBS + 1% BSA), adjusted to the appropriate concentration, and added to a 96-well plate at 1×10⁵ cells per well. Antibodies were then added sequentially according to the predetermined concentrations (8 concentrations starting from 200 nM with 4-fold serial dilutions), in which the negative control was human IgG1AA (Negative-IgG1AA, hereinafter referred to as NC, Biointron, Cat. No.: B 109802), and the anti-CD3 positive monoclonal antibody control molecule was h160c9-IgG1AA (hereinafter referred to as 160c9AA). After incubation on a shaker at 4°C for 2 hours, the cells were washed twice with FACS buffer by centrifugation, and fluorescently labeled anti-human IgG secondary antibody was added at 100 µL per well. After incubation on a shaker at 4°C for 0.5 hours, the cells were washed twice with FACS buffer by centrifugation, then filtered into a new 96-well plate, and the prepared samples were analyzed on a flow cytometer. The mean fluorescence intensity (hereinafter referred to as MFI) at each concentration was calculated by software, and the half maximal effective concentration (hereinafter referred to as EC₅₀) and the mean maximum fluorescence intensity (Top MFI) were calculated by GraphPad software. The results are shown in Table 7 as well as FIGs. 2 and 3.

**Table 7: Affinity of anti-CD3-GPC3 bispecific antibody for hGPC3 and hCD3**

| | HepG2 cells | | Jurkat cells | |
|---|---|---|---|---|
| | EC₅₀ (nM) | TOP (MFI) | EC₅₀ (nM) | TOP (MFI) |
| G100 | 0.42 | 3966 | 23.86 | 3505 |
| G156 | 0.16 | 1802 | 27.35 | 3300 |
| 160c9AA | / | / | 0.51 | 4271 |
| NC | / | / | / | / |

The results showed that the bispecific antibodies G100 and G156 exhibited concentration-dependent specific binding to HepG2 and Jurkat cells with good binding effects, while the affinity of the bispecific antibodies for human GPC3 was significantly higher than that for human CD3. Specifically, the EC₅₀ of G100 binding to human GPC3 was higher than that of G156, the binding affinity of G100 and G156 to human CD3 was comparable, and the binding capability of G100 and G156 to the hCD3 antigen was much weaker than that of the CD3 positive control antibody 160C9AA.

### B. In vitro binding affinity and kinetics of anti-CD3-GPC3 bispecific antibody to human/monkey CD3

In this example, the affinity and kinetic properties of the bispecific antibodies binding to human CD3 E&D protein (Acro, Cat. No.: CDD-H52W1) and monkey CD3 E&D protein (Acro, Cat. No.: CDD-C52W4) were determined by surface plasmon resonance (SPR) using a Biacore 8K instrument. Human/monkey CD3 E&D proteins were immobilized on the experimental flow cell of a CM5 chip via amino coupling. The antibodies to be tested, G100 and G156, were then diluted to 250 nM in 1×HBS-EP+ running buffer (10 mM HEPES, pH 7.4, 150 mM NaCl, 3 mM EDTA, 0.05% P20), respectively, followed by 2-fold serial dilution to 0.06 nM in the same buffer, resulting in antibody solutions in a concentration series ranging from 0.06 nM to 250 nM. The antibody solutions were sequentially injected over the experimental and reference flow cells for association, followed by dissociation, thus obtaining association and dissociation curves for each sample. The raw data were analyzed using Biacore Insight Evaluation Software (version 2.0.15.12933) with a 1:1 fitting model. The resulting affinity and kinetic experimental data of the bispecific antibodies are shown in Table 8.

**Table 8: Binding affinity and kinetics of anti-CD3-GPC3 bispecific antibody to human and monkey CD3 E&D proteins**

| Molecule number | Antigen | Association rate kₐ (1/M*s) | Dissociation rate k_{d} (1/s) | Affinity K_{D} (M) |
|---|---|---|---|---|
| G100 | Human CD3 E&D | 8.20E+05 | 2.86E-03 | 3.49E-09 |
| G156 | Human CD3 E&D | 1.48E+06 | 1.93E-03 | 1.30E-09 |
| G100 | Monkey CD3 E&D | 7.55E+05 | 4.27E-03 | 5.66E-09 |
| G156 | Monkey CD3 E&D | 1.32E+06 | 3.67E-03 | 2.79E-09 |

The results showed that the bispecific antibodies G100 and G156 exhibited high binding affinity for both human and monkey CD3 E&D proteins.

### C. In vitro binding affinity and kinetics of anti-CD3-GPC3 bispecific antibody to human/monkey GPC3

In this example, the affinity and kinetic properties of the bispecific antibodies binding to human GPC3 protein (Acro, Cat. No.: GP3-H52H4) and monkey GPC3 protein (Acro, Cat. No.: GP3-C522a) were determined by surface plasmon resonance (SPR) using a Biacore 8K instrument. Anti-human Fc antibody proteins were immobilized on the experimental flow cell of a CM5 chip via amino coupling. Human and monkey GPC3 proteins were then diluted to 250 nM in 1×HBS-EP+ running buffer (10 mM HEPES, pH 7.4, 150 mM NaCl, 3 mM EDTA, 0.05% P20), followed by 2-fold serial dilution to 1.95 nM in the same buffer, resulting in solutions in a concentration series ranging from 1.95 nM to 250 nM. The solutions were sequentially injected over the experimental and reference flow cells for association, followed by dissociation, thus obtaining association and dissociation curves for each sample. The raw data were analyzed using Biacore Insight Evaluation Software (version 2.0.15.12933) with a 1:1 fitting model. The resulting affinity and kinetic experimental data of the bispecific antibodies are shown in Table 9.

**Table 9: Binding affinity and kinetics of anti-CD3-GPC3 bispecific antibody to human and monkey GPC3 proteins**

| Molecule number | Antigen | Association rate kₐ (1/M*s) | Dissociation rate k_{d} (1/s) | Affinity K_{D} (M) |
|---|---|---|---|---|
| G100 | Human GPC3 | 1.33E+05 | 1.12E-03 | 8.36E-09 |
| G156 | Human GPC3 | 1.89E+05 | 1.02E-03 | 5.42E-09 |
| G100 | Monkey GPC3 | 3.02E+05 | 2.76E-03 | 9.15E-09 |
| G156 | Monkey GPC3 | 2.85E+05 | 2.75E-03 | 9.62E-09 |

The results showed that the bispecific antibodies G100 and G156 exhibited good and comparable binding affinity for both human and monkey GPC3 proteins, as well as comparable affinity between species.

### Example 4: In vitro functional assay of anti-CD3-GPC3 bispecific antibody

### A. T cell-dependent cellular cytotoxicity (TDCC) assay

With HepG2 cells as target cells and T cells isolated from healthy human PBMCs (peripheral blood mononuclear cells) as effector cells, the release of cellular lactate dehydrogenase (LDH) was measured using cytotoxicity assay reagent (Roche, Cat. No.: 04744934001), which was used as an indicator of cell killing.

HepG2 cells were collected by centrifugation, resuspended in cell buffer (EMEM + 10% FBS) after discarding the supernatant, adjusted to the appropriate density, transferred to a 96-well plate at 1×10⁴ cells per well with a volume of 100 µL, and incubated overnight. Following cell adherence, 50 µL of pre-prepared antibodies with different concentration gradients (8 concentrations starting from 100 nM with 8-fold serial dilutions) and control sample working solution were added, followed by 50 µL of pre-prepared T cells at a ratio of effector cells to target cells of 10:1, *i.e.,* 1×10⁵ cells per well. The plate was incubated in a cell incubator (37°C, 5% CO₂) for approximately 24 hours. After incubation, the 96-well assay plate was removed and centrifuged at 1500 rpm for 10 minutes. The supernatant was carefully pipetted, and 20 µL of cell lysis buffer was added to each well. Following complete lysis, the plate was centrifuged at 2000 rpm for 5 minutes, and 50 µL of cell supernatant was transferred to a new 96-well plate. 50 µL of LDH assay reagent was added according to the instructions of the LDH assay kit, and the plate was incubated at room temperature for 20 to 30 minutes. Absorbance was measured at a detection wavelength of 492 nm and a reference wavelength of 650 nm using a microplate reader.

The percentage of cell lysis induced by TDCC was calculated using the following formula: % Cell lysis = 100 × (sample release - target cell/effector cell release) / (maximum release - target cell release)

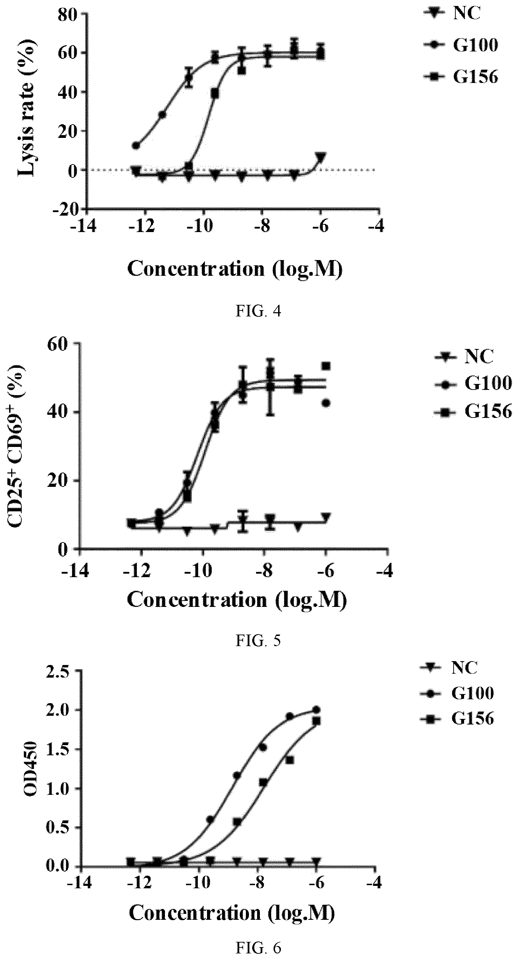

wherein the maximum release is the absorbance value generated in the wells containing target cells treated with Triton X-100; the target cell/effector cell release is the absorbance value generated in the wells containing a mixture of target cells and effector cells; the target cell release is the absorbance value generated in the wells containing only target cells; the sample release is the absorbance value generated in the wells containing a mixture of antibodies, target cells, and effector cells. EC₅₀ and maximum lysis were calculated by GraphPad software. The results are shown in Table 10 and FIG. 4.

### B. T cell activation assay

T cell activation was mainly identified by determining the cell activation marker CD25⁺CD69⁺%. After TDCC assay, cells were collected into a 96-well plate, washed twice with FACS buffer (PBS + 1% BSA) by centrifugation, and resuspended in 100 µL of FACS buffer. A certain amount of a mixture of anti-human BV421-CD25 (BD, Cat. No.: 562442) and anti-human PE-CD69 (BD, Cat. No.: 555531) was then added, and the plate was incubated at 4°C for 30 minutes. The cells were washed twice with FACS buffer by centrifugation, then filtered into a new 96-well plate, and the prepared samples were analyzed on a flow cytometer. The percentage of CD25⁺CD69⁺ cells in CD3⁺ T cells was calculated by software, and the half maximal effective concentration (hereinafter referred to as EC₅₀) and the highest percentage (CD25⁺CD69⁺%) were calculated by GraphPad software. The results are shown in Table 10 and FIG. 5.

**Table 10: TDCC activity and T cell activation of anti-CD3-GPC3 bispecific antibody**

| Molecule number | TDCC activity | | T cell activation | |
|---|---|---|---|---|
| | EC₅₀ (nM) | Maximum lysis (%) | EC₅₀ (nM) | CD25⁺CD69⁺% |
| G100 | 0.001 | 61.15% | 0.007 | 51.4% |
| G156 | 0.015 | 58.44% | 0.012 | 53.45% |
| NC | / | / | / | / |

The results showed that both bispecific antibodies G100 and G156 exhibited strong TDCC activity and T cell activation effects. Specifically, the TDCC activity of the bispecific antibody G100 was stronger than that of the bispecific antibody G156, while their T cell activation effects were comparable.

### C. Cytokine quantification assay

Cytokine quantification (including IL-2, TNF-α, and IFNγ) was mainly performed using ELISA Kits (BioLegend, Cat. Nos.: 431804, 430204, and 430104, respectively). After centrifugation, the supernatant was collected and diluted according to set ratios (1:8 dilution for IL-2 test samples, 1:5 dilution for TNF-α test samples, and 1:25 dilution for IFNγ test samples) before testing using the Kit. Absorbance was measured at a detection wavelength of 450 nm and a reference wavelength of 650 nm using a microplate reader. EC₅₀ was calculated by GraphPad software. The results are shown in Table 11. In addition, FIGs. 6 to 8 show the release of cytokines IL-2, IFNγ, and TNF-α when T cells freshly isolated from healthy human PBMCs (effector cells) and GPC3-positive HepG2 cells (target cells) were co-cultured in the presence of anti-CD3-GPC3 bispecific antibodies during the TDCC assay.

**Table 11: IL-2, TNF-α, and IFNγ secretion by anti-CD3-GPC3 bispecific antibody**

| Molecule number | IL-2 secretion | | TNF-α secretion | | IFNγ secretion | |
|---|---|---|---|---|---|---|
| | EC₅₀ (nM) | OD450 | EC₅₀ (nM) | OD450 | EC₅₀ (nM) | OD450 |
| G100 | 0.137 | 2.0 | 1.85 | 0.58 | 0.001 | 1.63 |
| G156 | 1.662 | 1.87 | 321 | 1.12 | 0.016 | 1.7 |
| NC | / | / | / | / | / | / |

The results showed that both bispecific antibodies G100 and G156 induced the release of IL-2, IFNγ, and TNF-α, with G100 exhibiting stronger cytokine release than G156.

### Example 5: In vivo efficacy assay of anti-CD3-GPC3 bispecific antibody

PBMCs were isolated and counted, and then co-cultured with Mitomycin C-treated HepG2 cells in RPMI-1640 medium (containing 10 ng/mL IL-2 and 10% FBS) for 6 days. After 6 days, PBMCs were collected and mixed with freshly digested HepG2 cells for subcutaneous inoculation into NCG mice (Shanghai LIDE Biotech Co., Ltd.) to construct a humanized HepG2 model. All experimental animals were housed in individually ventilated cages under constant temperature and humidity conditions: a temperature of 20.0 to 26.0°C, a humidity of 40 to 70%, a ventilation rate of 10 to 20 times/hour, and a light/dark cycle of 12 hours/12 hours.

In this experiment, mice were divided into three groups, namely PBS control group, 0.63 mg/kg G100 treatment group, and 1.89 mg/kg G100 treatment group, with 6 mice per group. The mice were administrated via intraperitoneal injection twice weekly (BIW) for a total of 6 doses (see Table 12). Animals were monitored daily for behavioral performance for 18 days after administration. Throughout the experiment, tumor length and width were measured twice weekly using a vernier caliper, with tumor volume (mm³) calculated as 0.5 × (tumor length × tumor width²). Relative tumor growth inhibition TGI (%) was calculated as: TGI% = (1 - T/C) × 100%. T/C% represents the relative tumor growth rate, *i.e.,* percentage of the relative tumor volume or tumor weight in the treatment group versus the PBS control group at a specific time point; T and C represent the tumor volume (TV) or tumor weight (TW) at a specific time point for the treatment group and the PBS control group, respectively. All data were expressed as Mean ± SEM, and Student's t-test was used to determine whether there was a significant difference in tumor volume and tumor weight between the treatment group and the control group, with p < 0.05 showing a significant difference.

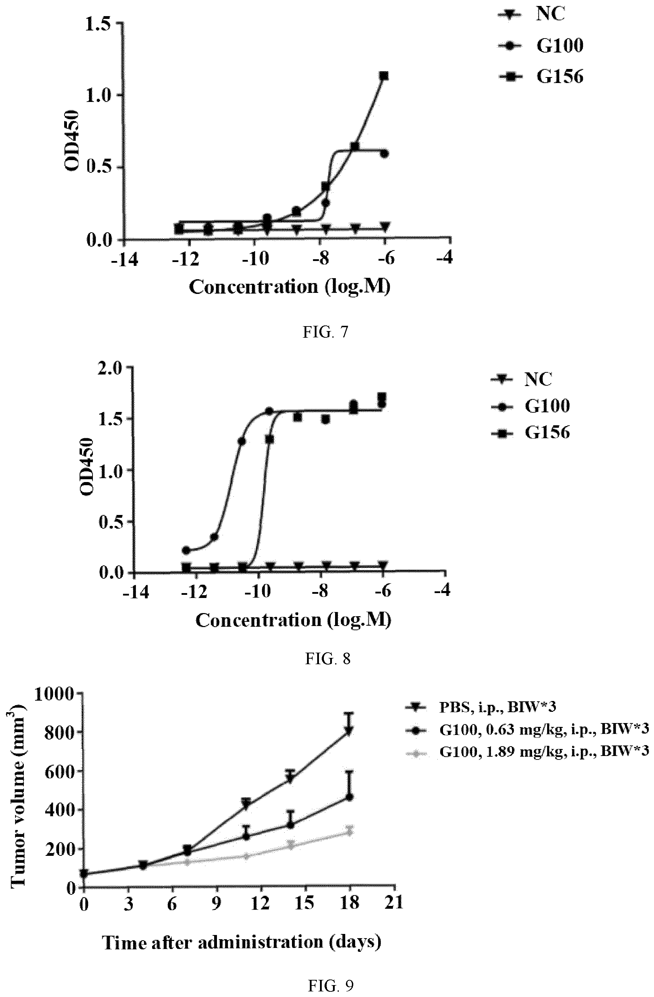

As shown in FIG. 9, in a subcutaneous xenograft model of human hepatocellular carcinoma HepG2 cells mixed with PBMCs, the bispecific antibody G100 demonstrated a good antitumor effect. Final mean tumor volumes for the three groups were 791.58 mm³ (PBS), 455.65 mm³ (0.63 mg/kg G100), and 271.79 mm³ (1.89 mg/kg G100), respectively, with corresponding tumor growth inhibition (TGI) values of 46.28% and 71.57% for the 0.63 mg/kg G100 and 1.89 mg/kg G100 groups, respectively.

Meanwhile, no significant weight loss in NCG mice was observed in any treatment group, indicating good tolerability of the anti-CD3-GPC3 bispecific antibody at these doses in NCG mice. These results demonstrate that the anti-CD3-GPC3 bispecific antibody exhibits excellent antitumor efficacy with favorable safety profile.

**Table 12: Experimental groups and dosing regimen**

| Group | Grouping | Dose (mg/kg) | Dosing regimen | Mode of administration |
|---|---|---|---|---|
| Group 1 | PBMC + control (PBS) | 0 | BIW*3 | Intraperitoneal administration |
| Group 2 | PBMC + G100 | 0.63 | BIW*3 | Intraperitoneal administration |
| Group 3 | PBMC + G100 | 1.89 | BIW*3 | Intraperitoneal administration |

The embodiments of the present disclosure described above are intended to be merely exemplary, and numerous equivalents of specific compounds, materials, and operations may be recognized or determined by one skilled in the art without undue experimentation. All such equivalents are intended to be within the scope of the present disclosure and are encompassed by the claims.

## Claims

1. A bispecific antigen-binding molecule, comprising a first antigen-binding domain that recognizes Glypican-3 (GPC3); and a second antigen-binding domain that recognizes a CD3 subunit of a T cell receptor; wherein the first antigen-binding domain comprises HCDR1, HCDR2, and HCDR3, which are derived from the heavy chain variable region as shown in SEQ ID NO: 10.

2. The bispecific antigen-binding molecule according to claim 1, wherein the first antigen-binding domain comprises HCDRs as shown in SEQ ID NOs: 11 to 13; preferably, the sequence of HCDR1 is as shown in SEQ ID NO: 11; the sequence of HCDR2 is as shown in SEQ ID NO: 12; and the sequence of HCDR3 is as shown in SEQ ID NO: 13.

3. The bispecific antigen-binding molecule according to claim 1 or 2, wherein the second antigen-binding domain comprises HCDR1, HCDR2, and HCDR3 derived from the heavy chain variable region as shown in SEQ ID NO: 1; and further comprises LCDR1, LCDR2, and LCDR3 derived from the light chain variable region as shown in SEQ ID NO: 2.

4. The bispecific antigen-binding molecule according to any one of the preceding claims, wherein the second antigen-binding domain comprises CDRs as shown in SEQ ID NOs: 4 to 9; preferably, the sequence of HCDR1 is as shown in SEQ ID NO: 4; the sequence of HCDR2 is as shown in SEQ ID NO: 5; the sequence of HCDR3 is as shown in SEQ ID NO: 6; the sequence of LCDR1 is as shown in SEQ ID NO: 7; the sequence of LCDR2 is as shown in SEQ ID NO: 8; and the sequence of LCDR3 is as shown in SEQ ID NO: 9.

5. The bispecific antigen-binding molecule according to any one of the preceding claims, further comprising one or more of the following features:
(1) the first antigen-binding domain is a nanobody, preferably a humanized camelid nanobody;
(2) the bispecific antigen-binding molecule comprises heavy chain constant regions CH2 and CH3, and lacks a light chain constant region and/or heavy chain constant region CH1; preferably, the heavy chain constant region comprises an Fc domain or a variant Fc; more preferably, the Fc is derived from mouse or human;
(3) the bispecific antigen-binding molecule comprises an Fc domain, wherein the Fc domain is an IgG Fc domain; preferably, the Fc domain is an IgG1 domain or an IgG4 domain;
(4) the bispecific antigen-binding molecule comprises an Fc domain, wherein the two polypeptide chains constituting the Fc domain have different sequences from each other; the Knob chain of the two polypeptides constituting the Fc domain comprises a mutation of amino acid residues to tryptophan at position 366 according to the EU numbering system; the Hole chain comprises a mutation of amino acid residues to serine at position 366, a mutation of amino acid residues to alanine at position 368, and a mutation of amino acid residues to valine at position 407 according to the EU numbering system; preferably, the Knob chain comprises S354C and T366W amino acid substitutions, and the Hole chain comprises Y349C, T366S, L368A, and Y407V amino acid substitutions; more preferably, the Hole chain further comprises an H435R substitution; further preferably, the Knob chain and/or Hole chain further comprises L234A and L235A substitutions; further preferably, the Knob chain and/or Hole chain further comprises a K447A substitution at the last position of the C-terminus;
(5) the bispecific antigen-binding molecule comprises an Fc domain, wherein the Fc domain comprises a Knob chain and a Hole chain, the sequence of the Knob chain being as shown in SEQ ID NO: 1 and the sequence of the Hole chain being as shown in SEQ ID NO: 15.

6. A bispecific antigen-binding molecule, comprising a first peptide chain and a second peptide chain, wherein the sequence of the first peptide chain is as shown in SEQ ID NO: 16 or 18, and/or the sequence of the second peptide chain is as shown in SEQ ID NO: 17.

7. A polynucleotide encoding the bispecific antigen-binding molecule or a fragment thereof according to any one of the preceding claims; or a recombinant vector comprising the isolated polynucleotide; or a host cell comprising the isolated polynucleotide or recombinant vector.

8. A method for producing the bispecific antigen-binding molecule according to any one of claims 1 to 6.

9. A pharmaceutical composition comprising: a pharmaceutically acceptable carrier, and one or more selected from the group consisting of the bispecific antigen-binding molecule according to any one of claims 1 to 6, the polynucleotide, the recombinant vector, and/or the host cell according to claim 7.

10. Use of the bispecific antigen-binding molecule according to any one of claims 1 to 6 or the pharmaceutical composition according to claim 9 in the manufacture of a medicament for treating and/or preventing a disease in an individual in need thereof; preferably, the disease is cancer; more preferably, the cancer is a GPC3-positive tumor; further preferably, the cancer is selected from hepatocellular carcinoma, gastric cancer, esophageal cancer, ovarian cancer, or non-squamous non-small cell lung cancer.

11. A method for treating and/or preventing a disease associated with GPC3 expression in a subject, or a method for preparing a pharmaceutical composition for treating and/or preventing a disease associated with GPC3 expression in a subject, comprising administering to the subject an effective amount of the bispecific antigen-binding molecule according to any one of claims 1 to 6 or the pharmaceutical composition according to claim 9; preferably, the disease associated with GPC3 expression is cancer; more preferably, the cancer is selected from hepatocellular carcinoma, gastric cancer, esophageal cancer, ovarian cancer, or non-squamous non-small cell lung cancer.
